# EUROPEAN PATENT SPECIFICATION

(11) **EP 1 634 618 B1**
(45) Date of publication and mention of the grant of the patent: **13.02.2008**
(21) Application number: 05014630.7
(22) Date of filing: 03.02.2003
(51) Int. Cl.: A61P 19/10, A61P 19/08, A61P 19/02

(54) **Method for identifying compounds that increase bone mineral density**
Methode zur Identifizierung von Verbindungen, die die Knochenmineral-Dichte erhöhen
Procédé servant à améliorer la densitè de minéraux osseux

(30) Priority: 08.02.2002 US 355255 P
(43) Date of publication of application: 15.03.2006
(62) Divisional of application: 03704519.2
(73) Proprietor: F. HOFFMANN-LA ROCHE AG, 4070 Basel (CH); The Government of the United States d.b.a. The Department of Veterans Affairs, Washington, DC 20420 (US); Oregon Health & Science University, Portland, OR 97201-3098 (US)
(72) Inventor: Allard, John David, Milpitas, CA 95305 (US); Klein, Robert Frederick, Portland, Oregon 97225 (US); Peltz, Gary Allen, Redwood City, CA 94062 (US)
(74) Representative: Witte, Hubert

(56) References cited:
- BOCAN T M A ET AL: "A specific 15-lipoxygenase inhibitor limits the progression and monocyte-macrophage enrichment of hypercholesterolemia-induced atherosclerosis in the rabbit" 1998, ATHEROSCLEROSIS, AMSTERDAM, NL, PAGE(S) 203-216 , XP002239131 ISSN: 0021-9150

## Description

The invention relates to a method for the identification of compounds that increase bone mineral density.

Lipoxygenases are nonheme iron-containing enzymes found in plants and animals that catalyze the oxygenation of certain polyunsaturated fatty acids, such as lipids and lipoproteins. Several different lipoxygenase enzymes are known, each having a characteristic oxidation action. Mammalian lipoxygenases are named by the position in arachidonic acid that is oxygenated. The enzyme 5-lipoxygenase converts arachidonic acid to 5-hydroperoxyeicosatetraenoic acid (5-HPETE). This is the first step in the metabolic pathway which yields 5-hydroxyeicosatetraenoic acid (5-HETE) and the leukotrienes (LTs). Similarly, 12- and 15-lipoxygenase convert arachidonic acid to 12- and 15-HPETE, respectively. Biochemical reduction of 12-HPETE leads to 12-HETE, while 15-HETE is the precursor of the class of compounds known as lipoxins.

A diverse array of biological effects are associated with the products of lipoxygenase activity, and many are implicated as mediators in various disease states. The C4 and D4 LTs are potent constrictors of human bronchial smooth muscle; LTB4 and 5-HETE, found in the synovial fluid of patients with rheumatoid arthritis, are potent chemotactic factors for inflammatory cells such as polymorphonuclear leukocytes (Green and Lambeth, Tetrahedron, 39, 1687 (1983)); 12-HETE has been found at high levels in the epidermal tissue of patients with psoriasis; the lipoxins have been shown to stimulate lysosomal enzyme and superoxide ion release from neutrophils. Thus, lipoxygenase enzymes play an important role in the biosynthesis of mediators of asthma, allergy, arthritis, psoriasis, and inflammation, and inhibitors of these enzymes interrupt the biochemical pathway involved in these disease states.

Human 15-lipoxygenase (15-LO) catalyzes the formation of 15-S-hydroxyeicosatetraenoic acid (15-S-HETE) from arachidonic acid (Kuhn and Borngraber, Lipoxygenases and Their Metabolites, Plenum Press, New York, (1999)). In mice, the synthesis of 15-S-HETE is carried out by 12/15-Lipoxygenase (Alox15), which is the murine homologue of human 15-LO. Murine 12/15-LO converts arachidonic acid to 12(S)-hydroxyeicosatetraenoic and 15-S-HETE in a 3:1 ratio, and can additionally convert linoleic acid to 13-hydroxyoctadecadienoic acid (13-HODE).

15-Lipoxygenase has previously been implicated in the pathogenesis of several diseases, including atherosclerosis (Harats et al., Arterioscler. Thromb. Vasc. Biol., 2100-2105, (2000)), asthma (Shannon et al., Am.Rev.Respir. Dis., 147, 1024-1028, (1993)), cancer (Shureiqi et al., JNCI, 92,1136-1142, (2000)), and glomerulonephritis (Montero and Badr, Exp. Neph., 8,14-19 (2000)). A number of classes of compounds have been identified that inhibit 15-lipoxygenase, including phenols, hydroxamic acids and acetylenic fatty acids (reviewed in Kuhn and Borngraber, Lipoxygenases and Their Metabolites, Plenum Press, New York, (1999)). The spectrum of inhibitory activities varies for these agents. For example, nordihydroguaiaretic acid has been shown to be an inhibitor of 5- and 15-lipoxygenase, naphthylhydroxamic acids have been shown to inhibit 5-, 12-, and 15-lipoxygenase (U.S. Patent No. 4,605,669), and a benzofluorene 15-lipoxygenase inhibitor, PD146176, has been reported to be relatively specific for the 15-lipoxygenase enzyme (Sendobry et al., Br. J. Pharm., 120, 1199-1206, (1997)). Evidence for 15-lipoxygenase involvement in atherosclerosis has come from studies with mice with a targeted deletion of Alox15 (Alox15 -/-). These Alox15 knockout mice were initially shown to have minor phenotypic differences including increased 5-LO activity (Sun and Funk, J. Biol. Chem., 271, 24055-24062, (1996)). However, disruption of Alox15 greatly diminished atherosceloritic lesions in apoE deficient (apoE -/-) atherosclerosis prone mice (Cyrus et al., J. Clin Invest., 103, 1597-1604, (1999)).

Although 5-LO has been implicated in the pathogenesis of several diseases, the biologic function of murine or human 15-lipoxygenase has not been determined with certainty, nor has a human clinical utility for inhibitors of 15-lipoxygenase been established. In particular, the utility of 15-lipoxygenase inhibitors for treatment of human osteoporosis and/or osteoarthritis has not previously been discovered.

Osteoporosis is caused by a reduction in bone mineral density in mature bone and results in fractures after minimal trauma. The disease is widespread and has a tremendous economic impact. The most common fractures occur in the vertebrae, distal radius and hip. An estimated one-third of the female population over age 65 will have vertebral fractures, caused in part by osteoporosis. Moreover, hip fractures are likely to occur in about one in every three woman and one in every six men by extreme old age.

Two distinct phases of bone loss have been identified. One is a slow, age-related process that occurs in both genders and begins at about age 35. This phase has a similar rate in both genders and results in losses of similar amounts of cortical and cancellous (spongy or lattice like) bone. Cortical bone predominates in the appendicular skeleton while cancellous bone is concentrated in the axial skeleton, particularly the vertebrae, as well as in the ends of long bones. Osteoporosis caused by age-related bone loss is known as Type II osteoporosis.

The other type of bone loss is accelerated, seen in postmenopausal women and is caused by estrogen deficiency. This phase results in a disproportionate loss of cancellous bone. Osteoporosis due to estrogen depletion is known as Type I osteoporosis. The main clinical manifestations of Type I osteoporosis are vertebral, hip and forearm fractures. The skeletal sites of these manifestations contain large amounts of trabecular bone. Bone turnover is usually high in Type I osteoporosis. Bone resorption is increased but there is inadequate compensatory bone formation. Osteoporosis has also been related to corticosteroid use, immobilization or extended bed rest, alcoholism, diabetes, gonadotoxic chemotherapy, hyperprolactinemia, anorexia nervosa, primary and secondary amenorrhea, transplant immunosuppression, and oophorectomy.

The mechanism by which bone is lost in osteoporosis is believed to involve an imbalance in the process by which the skeleton renews itself. This process has been termed bone remodeling. It occurs in a series of discrete pockets of activity. These pockets appear spontaneously within the bone matrix on a given bone surface as a site of bone resorption. Osteoclasts (bone dissolving or resorbing cells) are responsible for the resorption of a portion of bone of generally constant dimension. This resorption process is followed by the appearance of osteoblasts (bone forming cells) which then refill the cavity left by the osteoclasts with new bone.

In a healthy adult subject, osteoclasts and osteoblasts function so that bone formation and bone resorption are in balance. However, in osteoporosis an imbalance in the bone remodeling process develops which results in bone being replaced at a slower rate than it is being lost. Although this imbalance occurs to some extent in most individuals as they age, it is much more severe and occurs at a younger age in postmenopausal osteoporosis, following oophorectomy, or in iatrogenic situations such as those resulting from the use of corticosteroids or immunosuppressants.

Various approaches have been suggested for increasing bone mass in humans afflicted with osteoporosis, including administration of androgens, fluoride salts, and parathyroid hormone and modified versions of parathyroid hormone. It has also been suggested that bisphosphonates, calcitonin, calcium, 1,25-dihydroxy vitamin D₃, and/or estrogens, alone or in combination, may be useful for preserving existing bone mass.

The present invention is based on the discovery that inhibitors of 15-lipoxygenase are able to increase net bone formation and/or enhance bone accretion and enhance fracture healing. These molecules can be delivered alone or in combination with additional agents which inhibit bone resorption and/or enhance bone formation, particularly anabolic agents

The invention is directed to a method for identifying compounds that increase bone mineral density. The method comprises contacting a compound with 15-lipoxygenase and determining whether the compound inhibits 15-lipoxygenase activity.
These and other aspects of the present invention will become evident upon reference to the following detailed description and attached figures. The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B (1992).

In describing the present invention, the following terms will be employed, and are intended to be defined as indicated below.

By "bone loss" is meant an imbalance in the ratio of bone formation to bone resorption resulting in less bone than desirable in a patient. Bone loss may result from osteoporosis, osteotomy, periodontitis, or prosthetic loosening. Bone loss may also result from secondary osteoporosis which includes glucocorticoid-induced osteoporosis, hyperthyroidism-induced osteoporosis, immobilization-induced osteoporosis, heparin-induced osteoporosis or immunosuppressive-induced osteoporosis. Bone loss can be monitored, for example, using bone mineral density measurements described below.

By "increased bone accretion" is meant that bone accumulation in a subject administered the 15-lipoxygenase inhibitors of the invention is increased over bone accumulation in a comparable subject that is not given an 15-lipoxygenase inhibitor. Such increased bone accretion is typically determined herein by measuring bone mineral density (BMD). For example, bone accretion can be determined using an animal model, such as an ovariectomized mouse, dog and the like. The animal is administered the test compound and bone mineral density (BMD) measured in bones that are normally depleted in Type I or Type II osteoporosis, such as bones of the appendicular and/or axial skeleton, particularly the spine including the vertebrae, as well as in the ends of long bones, such as the femur, midradius and distal radius. Several methods for determining BMD are known in the art. For example, BMD measurements may be done usingdual energy x-ray absorptiometry or quantitative computed tomography, and the like. (See, the examples.) Similarly, increased bone formation can be determined using methods well known in the art. For example, dynamic measurements of bone formation rate (BFR) can be performed on tetracycline labeled cancellous bone from the lumbar spine and distal femur metaphysis using quantitative digitized morphometry (see, e.g., Ling et al., Endocrinology (1999) 140:5780-5788. Alternatively, bone formation markers, such as alkaline phosphatase activity and serum osteocalcin levels can be assessed to indirectly determine whether increased bone formation has occurred (see Looker et al., Osteoporosis International (2000) 11(6):467-480).

By "increased bone formation" is meant that the amount of bone formation in a subject administered the 15-lipoxygenase inhibitors of the invention is increased over the bone formation rate in a subject that is not given an 15-lipoxygenase inhibitor. Such enhanced bone formation is determined herein using, e.g., quantitative digitized morphometry, as well as by other markers of bone formation, as described above.

By "inhibitor of 15-lipoxygenase" is meant a compound that inhibits 15-lipoxygenase with an IC50 of less than 1µM, preferably less than 100nM. IC50's may be determined by standard methods. One particular method is a colorimetric assay in which the putative inhibitor is pre-incubated with the 15-lipoxygenase enzyme for about 10 minutes followed by addition of linoleic acid substrate for an additional 10 minutes. The product, 13-HPODE, is quantitated by coupling the reduction of the hydroperoxylated lipid to the oxidation on N-benzoyl-leucomethylene blue in the presence of hemin at pH 5. The absorbance of the oxidized methylene blue is directly proportional to the amount of 13-HPODE formed by the 15-lipoxygenase, and can be measured in the presence and absence of inhibitor. This is an endpoint assay described in more detail in "A Spectrophotometric Microtiter-Based Assay for the Detection of Hydroperoxy Derivatives of Linoleic Acid", Analytical Biochemistry, 201, 375-380 (1992); Bruce J. Auerbach, John S. Kiely and Joseph A. Cornicelli. Other assays include those where the initial enzyme reaction rate is measured spectrophotometrically by measuring conjugated diene formation at 234nm.

As used herein, the term "precursor cell" refers to a cell that is not fully differentiated or committed to a differentiation pathway, and that generally does not express markers or function as a mature, fully differentiated cell.

As used herein, the term "mesenchymal cells" or "mesenchymal stem cells" refers to pluripotent progenitor cells that are capable of dividing many times, and whose progeny will give rise to skeletal tissues, including cartilage, bone, tendon, ligament, marrow stroma and connective tissue (see A. Caplan J. Orthop. Res. (1991) 9:641-50).

As used herein, the term "osteogenic cells" includes osteoblasts and osteoblast precursor cells.

The compounds used in the present invention are those that inhibit or reduce the activity of 15-lipoxygenase. In this context, inhibition and reduction of the enzyme activity refers to a lower level of measured activity relative to a control experiment in which the enzyme, cell, or subject is not treated with the test compound. In particular embodiments, the inhibition or reduction in the measured activity is at least a 10% reduction or inhibition. One of skill in the art will appreciate that reduction or inhibition of the measured activity of at least 20%, 50%, 75%, 90% or 100% or any integer between 10% and 100%, may be preferred for particular applications. Typically, the 15-lipoxygenase inhibitors used in this invention will have IC50's of less than 1µM, preferably less than 100nM.

The present invention is based, in part, on the discovery that 15-lipoxygenase is an important modulator of bone mass. In particular, a genome scan of 100 microsatellite markers in mice led to the identification of bone loss disorder susceptibility loci on chromosomes 1, 2, 4, and 11. The differences in gene expression, especially for genes encoded on chromosome 11, identified substantial differences in expression of a gene encoding for 15-lipoxygenase, thereby linking the expression of 15-lipoxygenase to a lowered bone mineral density.

Compounds that inhibit 15-lipoxygenase activity and prevent bone resorption or promote bone formation provide important benefits to efforts at treating osteoporosis. Compounds that inhibit 15-lipoxygenase activity can be used in a method for treating osteoporosis or osteoarthritis by inhibition of osteoclastic bone resorption or by stimulation of osteoblast differentiation and promotion of new bone formation. Example 1 shows the ability of 15-lipoxygenase inhibitors to promote the differentiation of human mesenchymal stem cells into osteoblasts *in vitro.* Example 2 shows the ability of 15-lipoxygenase inhibitors to promote bone formation in an *in vivo* model.

Several methods for identifying classes of 15-lipoxygenase inhibitors that may also prevent bone loss and/or promote bone formation may be employed. One method used to identify compounds that inhibit 15-lipoxygenase activity involves placing cells, tissues, or preferably a cellular extract or other preparation containing 15-lipoxygenase in contact with several known concentrations of a test compound in a buffer compatible with 15-lipoxygenase activity. The level of 15-lipoxygenase activity for each concentration of test compound is measured by quantitation of enzyme product and the IC₅₀ (the concentration of the test compound at which the observed activity for a sample preparation is observed to fall one-half of its original or a control value) for the compound is determined using standard techniques. Other methods for determining the inhibitory concentration of a compound of the invention against 15-lipoxygenase are known to one of skill in the art and can be employed as will be apparent based on the disclosure herein. Specific assays that may be used to identify 15-lipoxygenase inhibitors include those described in U.S. Patent No. 6,268,387B1 and 5,958,950 (rabbit reticulocyte assay) and U.S. Patent No. 4,623,661 (radiolabelled arachidonic acid in RBL-1 cells)

For example, antagonists can normally be found once a ligand has been structurally defined. Testing of potential ligand analogs is now possible upon the development of highly automated assay methods using physiologically responsive cells. In particular, new agonists and antagonists will be discovered by using screening techniques described herein.

In another method, rational drug design, based upon structural studies of the molecular shapes of the chemokines, other effectors or analogs, or the receptors, may be used to identifying compounds whose three-dimensional structure is complementary to that of the active site of 15-lipoxygenase. These compounds may be determined by a variety of techniques including molecular mechanics calculations, molecular dynamics calculations, constrained molecular dynamics calculations in which the constraints are determined by NMR spectroscopy, distance geometry in which the distance matrix is partially determined by NMR spectroscopy, x-ray diffraction, or neutron diffraction techniques. In the case of all these techniques, the structure can be determined in the presence or absence of any ligands known to interact with 15-lipoxygenase.

Such computer programs include but are not limited to AMBER (available from University of California, San Francisco), CHARMM (Chemistry at HARvard Molecular Mechanics, available from Harvard University), MM2, SYBYL (Trypos Inc.), CHEMX (Chemical Design), MACROMODEL, GRID (Molecular Discovery Ltd), and Insight II (Accelryl). Such programs are contemplated as being useful for the determination of the chemical interaction between two molecules, either isolated, or surrounded by solvent molecules, such as water molecules, or using calculations that approximate the effect of solvating the interacting molecules. The relative orientation of the two can be determined manually, by visual inspection, or by using other computer programs which generate a large number of possible orientations. Examples of computer programs include but are not limited to DOCK and AutoDOCK. Each orientation can be tested for its degree of complementarity using the computer programs. Thus, novel compounds can be designed that are capable of inhibiting 15-lipoxygenase.

Another method for identifying compounds that may inhibit 15-lipoxygenase involves the use of techniques such as UV/VIS spectroscopy, polarimetry, CD or ORD spectroscopy, IR or Raman spectroscopy, NMR spectroscopy, fluorescence spectroscopy, HPLC, gel electrophoresis, capillary gel electrophoresis, dialysis, refractometry, conductometry, atomic force microscopy, polarography, dielectometry, calorimetry, solubility, EPR or mass spectroscopy. The application of these methods can be direct, in which the compound's interaction with 15-lipoxygenase is measured directly, or it can be indirect, in which a particular agent having a useful spectroscopic property is used as a probe for the ability of other compounds to bind to 15-lipoxygenase; for example, by displacement or by fluorescence quenching.

Accordingly, the present invention provides a method for identifying compounds that increase bone mineral density, the method comprising contacting a compound with 15-lipoxygenase and determining whether the compound inhibits 15-lipoxygenase activity. Preferably, said method further comprises testing the compound in a functional assay that demonstrates an effect of the compound on bone formation. More preferably, the functional assay comprises contacting the compound with human mesenchymal stem cells and determining cellular differentiation into bone forming cells. In another more preferred embodiment, the functional assay comprises administering the compound to a non-human animal and measuring an index of bone formation. In a most preferred embodiment, the index measured is bone mineral density. In another most preferred embodiment, the index is a biomechanical parameter of bone.

In another most preferred embodiment, the functional assay comprises contacting the compound with human mesenchymal stem cells and determining cellular differentiation into bone forming cells, wherein determining cellular differentiation comprises performing an alkaline phosphatase assay, a calcium assay, a total DNA preparation assay, or combinations thereof.

The 15-lipoxygenase inhibitors thus identified or designed can be subsequently tested for their ability to prevent bone loss and/or promote bone formation. In one embodiment, the computer based methods discussed above are used. In another method, the compounds are tested for their ability to modulate known targets for bone loss, such as, for example, estrogen receptors, tumor necrosis factor receptor, integrin receptor, and the like. In yet another method, the compounds are tested for their ability to differentiate stem cells into bone cells.

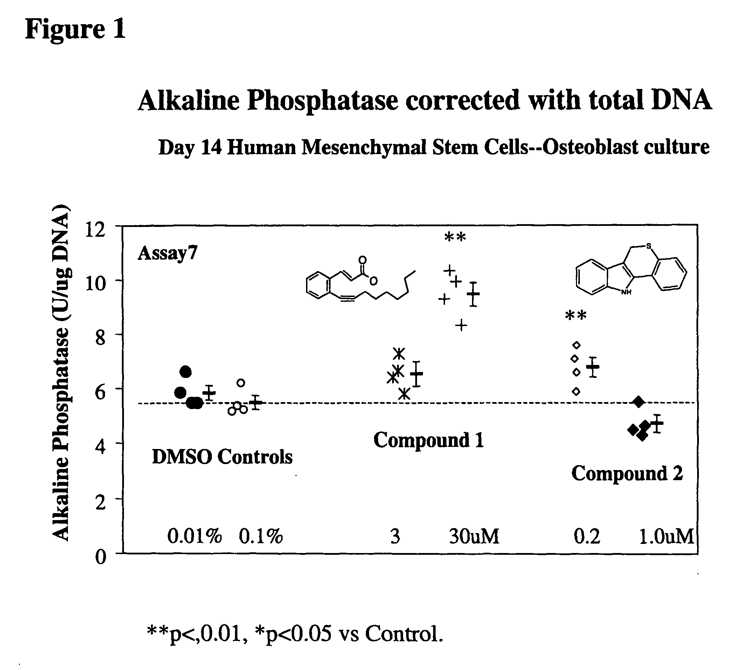

Figure 1 shows the quantitation of alkaline phosphatase activity in human mesenchymal stem cells (hMSC) in response to 15-lipoxygenase inhibitors Compound 1 or Compound 2 relative to solvent only (DMSO) control.

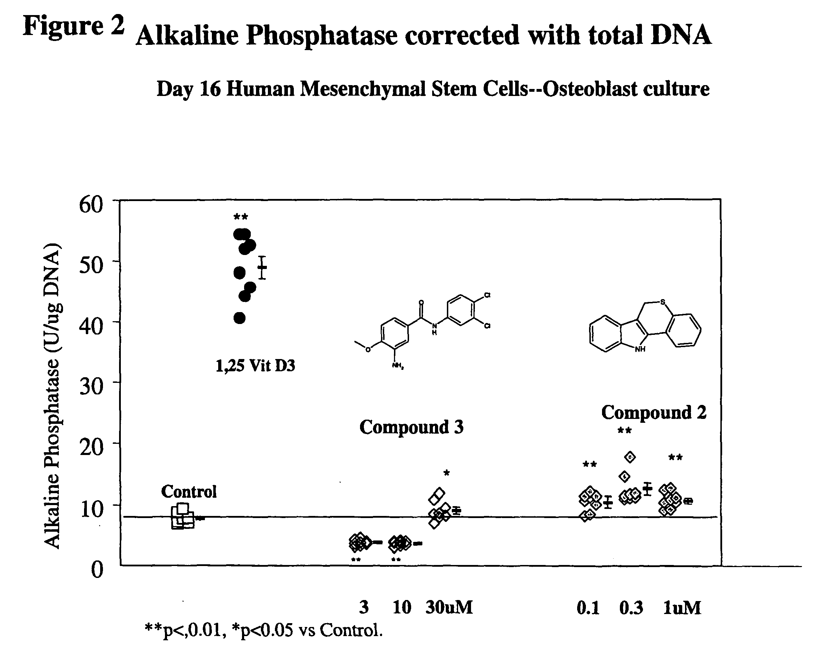

Figure 2 shows the quantitation of alkaline phosphatase activity in hMSC with Compound 3 or Compound 2 as 15-lipoxygenase inhibitors and DMSO or 1,25-Vitamin D₃ as the controls.

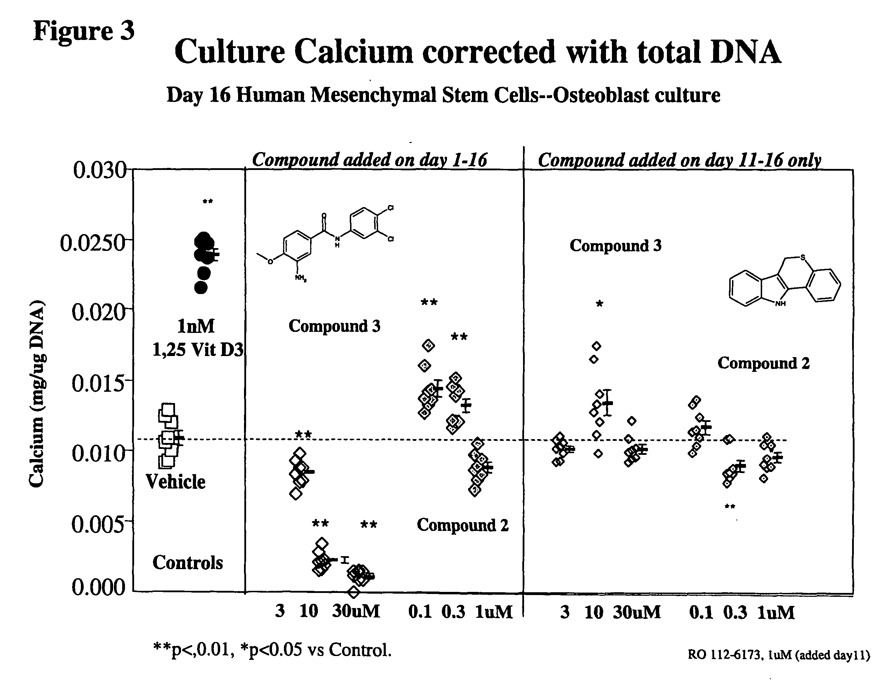

Figure 3 shows the quantitation of the total calcium content of hMSC with Compound 3 or Compound 2 as 15-lipoxygenase inhibitors and DMSO or 1,25-Vitamin D₃ as the controls.

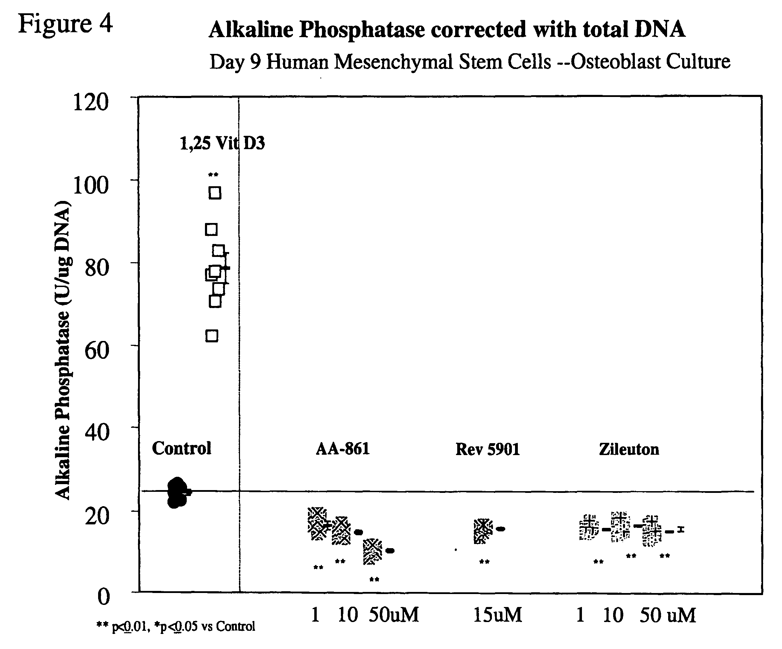

Figure 4 shows the quantitation of alkaline phosphatase activity in hMSC cultured for 9 days with the 5-LO inhibitors Zileuton, AA-861, and Rev-5901 relative to solvent only (DMSO) control. The lack of a response is in contrast to the effects seen with inhibitors of 15-lipoxygenase.
Figure 5 shows the quantitation of the total calcium content of hMSC cultured for 16 days with 5-LO inhibitors Zileuton, AA-861, and Rev-5901 relative to solvent only (DMSO) or 1,25-Vitamin D3 controls. The lack of a response is in contrast to the effects seen with inhibitors of 15-lipoxygenase.

Below are examples of specific embodiments for carrying out the present invention. The examples are offered for illustrative purposes only, and are not intended to limit the scope of the present invention in any way.

| Cpd. No. | Structure | IC50 for 15-LO |
|---|---|---|
| 1 | 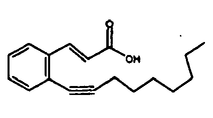 | 1-3 µM |
| 2 | 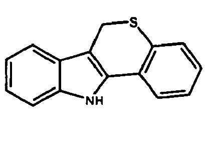 | ~ 200nM |
| 3 | 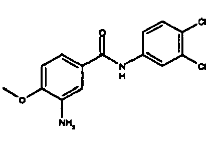 | ~10nM |
| 4 | 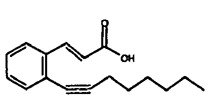 | 1-3µM |
| 5 | 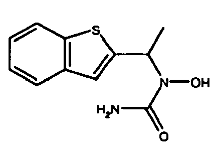 | negative control |
| 6 | 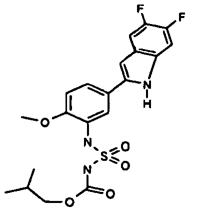 | 25-83nM |

Compound 1, 3-(2-non-1-ynylphenyl)-propionic acid, is described in U.S. Patent No. 5,972,980.

Compound 2, 6,11-dihydro-5-thia-11-aza-benzo[a]fluorene, is described in WO97/12613.

Compound 3, 3-amino-N-(3,4-dichlorophenyl)-4-methoxybenzamide, is described in WO99/32433.

Compound 4, *trans*-3-(2-oct-1-ynyl-phenyl)-acrylic acid is described in U.S. Patent No. 4,713,486.

Compound 5, Zilueton, is described in U.S. Patent No. 4,873,259.

Compound 6, [[[5-(5,6-difluoro-1H-indol-2-yl)-2-methoxyphenyl]amino]sulfonyl]-carbamic acid-isobutyl ester, is described in WO01/96298.

Efforts have been made to ensure accuracy with respect to numbers used (e.g., amounts, temperatures, etc.), but some experimental error and deviation should, of course, be allowed for.

### Example 1

### Ability of 15-lipoxygenase Inhibitors to Increase Bone Formation

In order to determine the ability of inhibitors of 15-lipoxygenase to stimulate bone formation and bone accretion, the ability of the 15-LO inhibitors to promote measures of differentiation of stem cells into bone forming cells (osteoblasts) was tested *in vitro.* In particular, the ability of three inhibitors, Compound 1 (3-(2-non-1-ynylphenyl)-propionic acid), Compound 2 (6,11-dihydro-5-thia-11-aza-benzo[a]fluorene), and Compound 3 (3-amino-N-(3,4-dichlorophenyl)-4-methoxybenzamide), prepared using literature procedures, to promote the differentiation of human mesenchymal stem cells into osteoblasts was determined by measuring two markers of osteoblast differentiation, cellular alkaline phosphatase activity and culture calcium content.

In general, human mesenchymal stem cells (hMSC, Poietics #PT-2501, BioWhittaker) were cultured in 12-well collagen coated culture plates at 3100 cells per square cm in 1 ml culture medium (Clonetics cat#PT-4105 plus osteoblast inducers (100nM Dex, 0.05mM L-Ascorbic acid-2-phosphate, 10 mM beta-glycerolphosphate). The three 15-LO inhibitors were added individually to each of the cultures, except for control wells to which was added 0.1% DMSO (negative control) or 1nM of 1,25-Vitamin D₃ (positive control). The concentrations of the inhibitors were as follows: Compound 1 at 3 and 30 µM; Compound 2 at 0.1, 0.2, 0.3, or 1.0µM and Compound 3 at 3, 10, or 30µM. Four to eight independent replicate cultures were prepared at each dose level. At the end of the experiments, the cultured cells were harvested by scraping the wells into the appropriate medium for further analysis of either alkaline phosphatase (Sigma kit#104-LL), or cellular calcium (Sigma kit#587-M). Cultures harvested for alkaline phosphatase assessment were harvested by scraping the cells and resuspending into 250µL Tris buffered 0.1% Triton X-100, and then assayed either fresh or following freeze-thaw. Separate cultures to be assayed for total calcium content were scraped and resuspended into 250µL 0.5M HCL. Results of these assays were normalized to total cellular DNA, thereby normalizing for differences in cell proliferation. At the same times that cultures were harvested for alkaline phosphatase and calcium, separate replicate cultures were harvested by scraping the cells and resuspending them in 250µL Hank's Balanced Salt Solution, and cellular number assessed by DNA (DNeasy Tissue Kit, Qiagen cat#69506).

### Quantitation of Osteoblast Differentiation in vitro.

For the quantitation of alkaline phosphatase activity, the inhibitors were prepared in 0.1% DMSO and added to the cultures on day 1 and every 2-3 days thereafter for 14-16 days. Three different sets of experiments were performed.

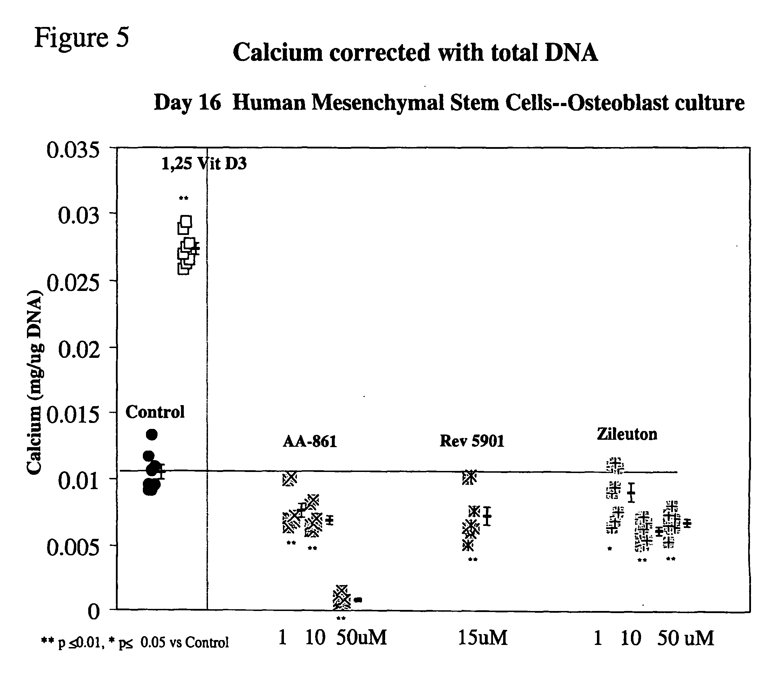

In the first experimental set, Compound 1 or Compound 2 (0.2µM or 1 µM) were used as inhibitors with the solvent (DMSO) as the negative control, and four independent replicate cultures were prepared at each dose level. The plates were cultured for 14 days. The DNA-normalized activity of alkaline phosphatase is plotted in Figure 5.

The second experimental set was comprised of eight replicate cultures with Compound 3 (3, 10, and 30µM) or Compound 2 (0.1, 0.3, 1.0µM) as the 15-LO inhibitors. Inhibitors were added either on Day 1 or Day 11. DMSO was used as the negative control, and 1,25-Vitamin D3 as the positive control for osteoblast differentiation induction. The normalized activity of alkaline phosphatase and calcium content is plotted in Figures 6 and 7, respectively. Compound 2 was most effective when added on Day 1, and Compound 3 was most effective when added on Day 11.

As a control for the specificity of 15-LO inhibition for the induction of osteoblast differentiation of stem cells in vitro, the third set of experiments studied eight replicates of standard lipoxygenase inhibitors recognized as more specific to the 5-LO enzyme, rather than the 15-LO enzyme. Zileuton (1,10, 50uM, prepared at Roche as) Compound 5, AA-861 (1, 10, 50uM, Sigma # A3711) and Rev-5901 (15uM, Sigma #R5523) were prepared fresh every 2-3 days and tested in the human osteoblast precursor cells. The 5-LO inhibitors were found to lack ability to induce alkaline phosphatase activity on day 9 or culture calcium deposition after 16 days of culture, as seen in Figures 8 and 9, respectively.

The results from the quantitation of markers of osteoblast differentiation, both alkaline phosphatase activity and culture calcium content, in hMSC cultures stimulated to differentiate into osteoblasts demonstrate that addition of specific 15-lioxygenase inhibitors promotes differentiation of the human bone forming cells *in vitro.*

In addition, the ability of 15-lipoxygenase inhibitors to stimulate bone formation and bone accretion is measured by the use of clonogenic assays where the clonogenic potential of the bone marrow precursor cells is measured. The osteoblast and osteoclast cells are treated with 15-lipoxygenase inhibitors. In one method, the cells and the inhibitors are incubated *in vitro.* In the another method, the mammal is administered the inhibitor, the bone marrow precursors are isolated, and then plated. For both methods, the colony forming units derived from these marrow cells is measured. One can screen for compounds with the potential for increasing bone mineral density if they are can be shown to increase the osteoblast clonogeneic potential of the marrow or decrease the osteoclast clonogeneic potential of the marrow.

### Example 2

### 15-Lipoxygenase Inhibitors promote bone formation in vivo

The ability of 15-lipoxygenase inhibitors to promote bone formation *in vivo* was assessed by measuring the ability of the inhibitors to improve bone parameters in a mouse model of osteoporosis. Constitutive expression of an Interleukin-4 transgene from the Lck gene promoter (Lck-IL4) in C57BL/6 mice results in a severe bone phenotype mimicking osteoporosis. (Lewis et al., Proc. Natl. Acad. Sci., 90, 11618-22, (1993)) and these mice were used to demonstrate the effect of treatment with 15-LO inhibitors on bone mass.

The four experimental groups used are shown in Table 2. At the time of weaning, wild type C57BL/6 or transgenic Lck-IL4 mice received 150mg/kg bid daily of Compound 2 PD146176 in their food for 84 days (Diet 5001: 23% protein, 10% fat, 0.95% calcium, and 0.67% phosphorus; PMI Feeds, Inc., St. Louis, MO). Mice were permitted access to one-half the daily food intake twice daily at approximately 12 h intervals and diet intake was monitored daily. Water was available ad libitum. The control groups received the same diet without Compound 2. All groups consumed food equally well over the course of the experiment.

**Table 2.**

| C57BL/6 | | Lck-IL4 transgenic | |
|---|---|---|---|
| Control | Compound 2 | Control | Compound 2 |
| n=17 | n=18 | n=20 | n=22 |
| (8F/9M) | (9F/9M) | (11F/9M) | (11F/11M) |

| | | | |
|---|---|---|---|
| Table 2. Experimental groups used for in vivo inhibitor studies. After weaning (approximately 28 days), mice were fed chow with or without 15-LO inhibitor (Compound 2) for 84 days. F, female and M, male animals were equally represented among the groups. | | | |

The effects of IL4 overexpression and 15-LO inhibition on whole body parameters, femoral BMD and geometry, and femoral biomechanics are shown in Table 3. Overexpression of IL4 results in a significant decrease in whole body BMD, body weight, and hematocrit and an increase in percent body fat. Overexpression of IL4 also reduced femoral BMD, cortical area, moment of inertia, and cortical thickness compared to wild type C57BL/6 mice. The increase in marrow area in Lck-IL4 mice correlates with the decreased hematocrit and associated anemia that has been reported. Additionally, IL4 overexpression had a significant effect on femoral biomechanics, including decreased failure load, bone stiffness and bone strength (Table 3). The mice that were fed the 15-LO inhibitor (Compound 2) showed partial reversal of the deleterious effects on whole body and femoral bone (Table 3). The drug-treated mice had a significant increase in whole body BMD, hematocrit, femoral BMD, and cortical thickness relative to control mice carrying the Lck-IL4 transgene. Importantly, the 15-LO inhibitor treated mice had a significant increase in femoral biomechanics, including failure load, stiffness and strength. This is particularly relevant to osteoporosis in that the level of bone strength and failure load are significant determinants in patients that go on to develop fractures. In summary, the results indicate that inhibition of 15-LO *in vivo* leads to a higher BMD and higher bone strength in an animal model of osteoporosis.

**Table 3.**

| Whole Body | | | | |
|---|---|---|---|---|
| Phenotype | Effect of IL-4 overexpression | p value | Effect of IL-4 overexpression + Compound 2 | p value |
| Body Weight | -7% | 0.042 | NC | ns |
| % Body Fat | +19% | 0.007 | NC | ns |
| Hematocrit | -25% | 2 x 10⁻⁹ | +20% | 2 x 10-5 |
| Whole Body BMD | -11% | 2 x 10⁻⁹ | +5% | 7 x 10-5 |
| | | | | |

| Femoral BMD & Geometry | | | | |
|---|---|---|---|---|
| Phenotype | Effect of IL-4 overexpression | p value | Effect of IL-4 overexpression + Compound 2 | p value |
| BMD | -17% | 4 x 10⁻¹² | +9% | 3 x 10⁻⁶ |
| Length | NC | ns | NC | ns |
| Total area | NC | ns | -4% | 0.035 |
| Cortical area | -25% | 6 x 10⁻¹² | NC | ns |
| Marrow area | +16% | 4 x 10⁻⁷ | - 7% | 7 x 10⁻⁵ |
| Moment of Inertia | -17% | 7x10⁻⁵ | NC | ns |
| Cortical thickness | -27% | 9x10⁻¹⁰ | +6% | 0.037 |
| | | | | |

| Femoral Biomechanics | | | | |
|---|---|---|---|---|
| Phenotype | Effect of IL-4 overexpression | p value | Effect of IL-4 overexpression + Compound 2 | p value |
| Failure load | -45% | 6 x 10⁻¹³ | +18% | 0.003 |
| Stiffness | -48% | 3 x 10⁻¹¹ | +11% | 0.09 |
| Strength | -34% | 2 x 10⁻¹² | +22% | 2 x 10⁻⁴ |

Table 3. Effects of IL4 overexpression and 15-LO inhibitor on whole body and femoral bone parameters. Whole body and femoral bone parameters were measured in mice carrying the Lck-IL4 transgene that leads to overexpression of IL4 compared to C57BL/6 controls. These parameters were also measured in Lck-IL4 mice that were fed the 15-LO inhibitor Compound 2 compared to untreated Lck-IL4 controls. NC, Not Changed between groups. ns, p-value was not significant.

### Animals

All mice used in the *in vivo* experiments were bred under identical conditions at the Portland VA Veterinary Medical Unit from stock originally obtained from The Jackson Laboratory (Bar Harbor, ME). Breeding mice were maintained for no more than three generations from stock obtained from The Jackson Laboratory. At the time of weaning the mice were group housed (9-10 animals per cage) in a 12 hr light/dark cycle (6:00 AM to 6:00 PM) at 21 ± 2°C. All procedures were approved by the VA Institutional Animal Care and Use Committee and performed in accordance with National Institutes of Health guidelines for the care and use of animals in research.

### Bone densitometry.

Bone mineral measurements were determined by dual energy X-ray absorptiometry (DEXA). All studies were performed with a Lunar PIXImus densitometer (Lunar Corp., Madison, WI). Whole body densitometric analyses were performed on anesthetized mice that were 4 months of age when the acquisition of adult bone mass is complete. The global window was defined as the whole body image minus the calvarium, mandible, and teeth. After sacrifice, the right femora were carefully removed and cleaned of adhering tissue prior to DEXA scanning.

### Specimen Processing

Adult mice (16 weeks old) were euthanized by CO₂ inhalation and weighed to the nearest 0.1 gm. Cardiac blood draws were obtained immediately upon sacrifice and a small aliquot of whole blood was reserved for hematocrit determination. Lumbar vertebrae and both femora were immediately harvested, wrapped in sterile gauze soaked in phosphate-buffered saline, and stored frozen at less than about -20 °C for subsequent analyses.

### Femoral Shaft Geometry

The cross-sectional geometric parameters of the mid-diaphysis of the femora were determined using a portable x-ray microtomograph (Model 1074, Skyscan, Antwerp, Belgium). Scans were taken at 40mm intervals, and a slice located at the midpoint of the femur was analyzed for total area (FCSA), cortical area (Ct Ar) and medullary canal area (Ma Ar). In addition, using the pixels contained in the cortical region of the digital image, the radius from the centroid of the cross-section to the outer fiber in the anterior-posterior plane, the areal moment of inertia (Ixx) in the plane of bending and the average cortical thickness (Ct Th) were calculated.

### Biomechanical Studies

The femur was tested to failure in three-point bending on a high resolution materials test apparatus (Instron Model 4442, Canton, MA). Failure load and stiffness were determined using system software. Bending strength was then calculated using the cross-sectional areal measurements previously determined.

### Data Analysis

All data were analyzed by a two-way analysis of variance (ANOVA) using the JMP software package (SAS Institute).

### Example 3

### Ability of a 15-lipoxygenase Inhibitor to Increase Bone Formation In Vivo

In order to determine the ability of inhibitors of 15-lipoxygenase to stimulate bone formation and bone accretion, [[[5-(5,6-difluoro-1H-indol-2-yl)-2-methoxyphenyl]amino]sulfonyl]-carbamic acid-isobutyl ester, Compound 6, was prepared as described in WO0196298) and tested in the standard estrogen deficiency Rat OVX osteopenia assay.

Three month old rats are ovariectomized (Ovx) and administered 1mg/kg/day Compound 6 or 0.1µg/kg/day 1,25-dihydroxy vitamin D₃ (positive control, abbreviated Vit. D in Table) by oral gavage once a day starting at 2 weeks post-ovariectomy and continuing for 7 weeks. The dose was increased to 2 mg/kg/day and continued until 11 weeks. Control groups, both sham (rats that were not ovariectomized) and Ovx, received vehicle only. The bone mineral density of the spine and right hip was determined by using the High Resolution Software package on a QDR-4500 Bone Densitometer (Hologic, Walthan, MA). The animals were scanned by placing them in a supine position such that the right femur was perpendicular to the main body and the tibia was perpendicular to the femur. The bone mineral density (g mineral/cm²) for the compounds at the hip and spine are given in Table 4 below. Animals treated with the 15-LO inhibitor showed increased BMD in the spine at >3 weeks and the proximal femur at > 7 weeks. Results are shown in Table 4.

**Table 4.**

| Treatment duration | Surgery Treatment Dose | | | BMD | | |
|---|---|---|---|---|---|---|
| | µg/kg/day | | | Lumbar Spine | | Proximal Femur |
| | | | | L2-L4 | L5 | |
| 3 weeks | Sham | Vehicle | | nd | nd | nd |
| | Ovx | Vehicle | | 0.2224 | 0.2343 | 0.2737 |
| | Ovx | Vit D | 0.1 | nd | nd | nd |
| | Ovx | **Cpd 6** | 1000 | 0.2347 *(0.055)* | 0.2480 *(0.041)* | 0.2838 *(0.103)* |
| 7 weeks | Sham | Vehicle | | .2664 | 0.2841 | 0.3068 |
| | Ovx | Vehicle | | 0.2303 | 0.2387 | 0.2829 |
| | Ovx | Vit D | 0.1 | 0.2628 *(0.023)* | 0.2742 *(0.015)* | nd |
| | Ovx | Cpd 6 | 1000 | 0.2474 *(0.019)* | 0.2549 *(0.120)* | 0.2932 *(0.033)* |
| 11 weeks | Sham | Vehicle | | 0.2740 | 0.2895 | 0.3177 |
| | Ovx | Vehicle | | 0.2353 | 0.2525 | 0.2842 |
| | Ovx | Cpd 6 | 2000 | 0.2507 *(0.009)* | 0.2626 *(0.059)* | 0.2986 *(0.039)* |

| | | | | | | |
|---|---|---|---|---|---|---|
| Values in parentheses are p values vs OVX control at the same timepoint. nd = no data | | | | | | |

## Claims

1. A method for identifying compounds that increase bone mineral density, the method comprising contacting a compound with 15-lipoxygenase and determining whether the compound inhibits 15-lipoxygenase activity.

2. The method of Claim 1, further comprising testing the compound in a functional assay that demonstrates an effect of the compound on bone formation.

3. The method of Claim 2, wherein the functional assay comprises contacting the compound with human mesenchymal stem cells which are not of human embryonic origin and determining cellular differentiation into bone forming cells.

4. The method of Claim 2, wherein the functional assay comprises administering the compound to a non-human animal and measuring an index of bone formation.

5. The method of Claim 4, wherein the index measured is bone mineral density.

6. The method of Claim 4, wherein the index is a biomechanical parameter of bone.

7. The method of claim 3, wherein determining cellular differentiation comprises performing an alkaline phosphatase assay, a calcium assay, a total DNA preparation assay, or combinations thereof.

## Patentansprüche

1. Verfahren zur Identifizierung von Verbindungen, die die Knochenmineraldichte erhöhen, wobei das Verfahren das Kontaktieren einer Verbindung mit 15-Lipoxygenase und das Bestimmen, ob die Verbindung die 15-Lipoxygenaseaktivität inhibiert, umfaßt.

2. Verfahren nach Anspruch 1, das ferner das Testen der Verbindung in einem funktionellen Assay, der die Wirkung der Verbindung auf die Knochenbildung zeigt, umfaßt.

3. Verfahren nach Anspruch 2, wobei der funktionelle Assay das Kontaktieren der Verbindung mit menschlichen Mesenchymstammzellen, die nicht menschlichen embryonalen Ursprungs sind, und das Bestimmen der Zelldifferenzierung zu knochenbildenden Zellen umfaßt.

4. Verfahren nach Anspruch 2, wobei der funktionelle Assay das Verabreichen der Verbindung an ein nicht menschliches Lebewesen und das Messen eines Anzeichens für Knochenbildung umfaßt.

5. Verfahren nach Anspruch 4, wobei das gemessene Anzeichen die Knochenmineraldichte ist.

6. Verfahren nach Anspruch 4, wobei das Anzeichen ein biomechanischer Parameter des Knochens ist.

7. Verfahren nach Anspruch 3, wobei die Bestimmung der Zelldifferenzierung die Durchführung eines alkalische Phosphatase-Assays, eines Cälcium-Assays, eines Gesamt-DNA-Präparations-Assays oder Kombinationen davon umfaßt.

## Revendications

1. Procédé pour identifier des composés qui augmentent la densité minérale osseuse, le procédé comprenant la mise en contact d'un composé avec la 15-lipoxygénase et déterminer si le composé inhibe l'activité de la 15-lipoxygénase.

2. Procédé selon la revendication 1, comprenant en outre le test du composé dans une analyse fonctionnelle qui démontre un effet du composé sur la formation osseuse.

3. Procédé selon la revendication 2, dans lequel l'analyse fonctionnelle comprend la mise en contact du composé avec des cellules souches du mésenchyme qui ne sont pas d'origine embryonnaire humain et la détermination de la différenciation cellulaire dans les cellules de formation osseuse.

4. Procédé selon la revendication 2, dans lequel l'analyse fonctionnelle comprend l'administration du composé à un animal non-humain et la mesure d'un indice de formation osseuse.

5. Procédé selon la revendication 4, dans lequel l'indice mesuré est la densité minérale osseuse.

6. Procédé selon la revendication 4, dans lequel l'indice est un paramètre biomécanique de l'os.

7. Procédé selon la revendication 3, dans lequel la détermination de la différenciation cellulaire comprend la conduite d'une analyse de la phosphatase alcaline, une analyse du calcium, une analyse de préparation ADN totale ou leurs combinaisons.


### SEQUENCE LISTING

<110> F. Hoffmann-La Roche AG
<120> Method of treating and preventing bone loss
<130> Case 21150
<150> US60/355255
<151> 2002-02-08
<160> 2
<170> Patent In version 3.1
<210> 1
   <211> 7864
   <212> DNA
   <213> Mus musculus
<220>
   <221> Ensembl gene ENSMUSG00000018924
   <222> (1)..(7864)
   <223>
<220>
   <221> position 70929619 in mouse genome
   <222> (1)..(1)
   <223>
<220>
   <221> position 70937482 in mouse genome
   <222> (7864)..(7864)
   <223>
<400> 1
   tctttgcaac attttattta aaataattag gtccctgttc ttttctatca ctagcccaaa      60
   acatctttta ttgttgctat tagactctat taaggcttga gatctatttg attcatagac     120
   gatgtgtaac aatgtatatt atcacccttt tgtgttcccg gggtgctttt cccaagcatg     180
   gctattattt accctatggg gaaggaaaag cagaaaggat gaatgaaaag tggcactctg     240
   ggtaacgcat ccaaagcaaa atgtgttcac tacagcagac tatggaaagc gggctcttgg     300
   aaaagggttc cacactggca gagggcgggg ctcaagcaca gggatgcact tgagtggtct     360
   tgattaaata acaaatcgag aggggggatg gtcatatggc cacgctgttt tctaccaggc     420
   tgggccgcag gtactcataa ggtatgtcca agctcttgtt acgaatctca atttccttat     480
   ccaaggcagc cagctcctct ctgaacttct tcagcacagc tttggcctca gggtttggga     540
   agtgttcctc tgaatgctgg cccaggggca cctggaaggc aggtgggaaa acagcctaag     600
   cctgtgacct ccgccagttt tggggtgcag agtttcctct ggaccctgaa tgattgattg     660
   actctcacca taacagcctg gcgtctgccc aggagccaaa cgacatttat ctggagagta     720
   gactgattag gattgggcag cgtcgccatc agcttctcca tcgtcgcgtc cttggtttta     780
   ggtggtggca gccgcatggt gcagggtgca ttaggaaccc agtagaacca atccagctgg     840
   agagacagtg atctagggtc aaggctcgcc cttggccccc atgctcacat tcccctcccc     900
   cactgtactc cccaaattcc cagctgcctc cctctgggaa gtacctggcc aagatggatg     960
   gaagagtgct gcgcagtgca tgtgaagatg cacatggtga tgaagtggca agcctgagcc    1020
   cgggactgga gggaggtggg gaagcctacg agaggtgtga acggttagcg tgtgacgtca    1080
   ccagaaccac tagacgggag gcctggagag gagagggaga ggcgagaagg acctcccagg    1140
   accttgtgta agctggtatg agagcgggga tggtgaggga cgagaggagc cctggcacct    1200
   atgtcataca taccccatcc ccaaggctca gggatcattg tcaaagggat gacagaaaaa    1260
   acttttgaga gttttcatga agatttaaat gcttctggga gagtccatgg aagcacagag    1320
   ggttctggag acgtctcaga actcaggtct tagttctcag gccagggatt cttattacct 1380
   ctgtcctggg caccttgcaa cccaatctca gtgatctctt gacaccagct ctgcagttca 1440
   tagtcatcct gaacagcttg gtcggtcttg tagtagagat cgaacattcc caccacgtac 1500
   ctgtcaccaa agggtcaggg caagctgagg ccctctgctc cctgctccat gtggctgagt 1560
   catagtgtcc acacccaacc aagccaagga acagacctcc tattctcctg gtcttctcca 1620
   cccacaccgg cacacaaacc cgtcctgcgg ctctgttgac ctcaccgatt catgacttgc 1680
   cagagctgca gggcatcttt agcatagaag caagtgtcaa tatccaggag tcctcgctca 1740
   gccaagtcat cagggggaca caatgagctg taggtcagga aggctccagc ttgcttgaga 1800
   agatccaggt ggcctcctcc acctgtgctc atcacctaaa ggaccaaggg caggtggttt 1860
   tcactctaat caagctgccc tgaagggaga ggacagagtg aacctagagc ccacttctat 1920
   ccccatacct tgtcaaaaaa gcctctctct gagatcaggt cgctcctggc ccggacattg 1980
   atttccatgg tgtagagtag gtgaggaact agaagctagg agaagaaaag ggatgggtaa 2040
   gtcagaagga ctctatgaca ggtccctgta gcaagcaggt ttgacaaggg agaagcgaag 2100
   ctcactccaa gggaaaacgt ggccgtgagg ctctgtggcc ctcgattctg ataagtgctg 2160
   gttttctgcc tgactggggc cacaagagtc aaatcactgc cttctgacaa tctctttcat 2220
   tccctgtgtt gtcttttcaa cagtctttct tgatcaccca aaagaaactc acatggcact 2280
   gttgtgattc tctaagatct caccacagcc aacatgtcga gtataatcac tactggggtt 2340
   tctgttgtta tttttgtttt gctttgtttg tttttttttt cctcttactg gagataaaaa 2400
   aaaaatcccc caaaactaga tgttaacaca ggcatctaga ctaggtctgt gtagagacat 2460
   ccaataccaa tttcgatgga tgaactcttt gctctgattg actagagtaa accaggtcac 2520
   tcttgttgct ctaggtcatc gtttcagaac tccccaaaga ctgctgtaag gaatccgttg 2580
   tgtaatttgc atggtcccct ctttaagtca cgtgcaccta ctctggtact gtatgtatat 2640
   ctatgttctg tacataagat tagaatcttt acaccctttg ctgaggaatc acctgtatat 2700
   gcccccttgg ggattagtgc cattgctgac aatgtagagg acagcaggtc acactccctt 2760
   tctctctccg ttgttcatct catctcttct catcatcctg aaccctgcct gagatgccac 2820
   tgagagaccg tctcagaggc aagagtgtgt agcaagccaa gagatgtaga cagaacaaaa 2880
   aaaaacacaa aaaacaaaaa acaaacaaac aaaaaaaaac cgaataaggt gttaccttaa 2940
   aaacagggtg cacggaaggc aggcacctca tggtggccac agcaaagacc tcagccacca 3000
   agtgtcccct cagaagatga gcctgtagct catgaagctg taagtctgag cttcggaccc 3060
   agcatttggc caggagccag tccattgggg gatccaaggg cgtgaaaatc ggcggtgggg 3120
   tagacccagt tttgggcagt tcgagctgga gtaaaacaac agtgtgaaga aagggctgga 3180
   tggagatctt cgtggcaacg gctcagcgat ccctacttcc tccatcccac acgtgtgcag 3240
   ccatcttggg ctctctctca tctttcaaca tcacctctcc acaaagatct gaggctctgg 3300
   gtttcaacta atagaggatt ttgtatgtgt ccccattact tcaaaactgg caggaccaaa 3360
   gcctacctgg gtgcctaccc ctctgcactt gccctgtgtc tgaagaaacc tgcctgagtc 3420
   ccacttttcc tgactccact ccaggccagt agattctccc tctgctctcc ctcctctcca 3480
   gctccatggc tacttactac tcacggagag cccaccatcc cctgaaacct tagcaacccg 3540
   gcatcagtaa gccccggcta tccattctaa cactgactct ggagaggagc tgctcacgct 3600
   gcgcctccag caagccacag aaatctcctg gctcctccac ctcagcctct tcacctaaag 3660
   tatctttctc tggggacaga tcccacccat cctcccgaca ctgctcccag gatcccccct 3720
   gaactggctc tgtccttggc attggagctt atacatgccg attagtctgt ctctccagta 3780
   aagccacaaa acccttaaag tcaaagcctg cccgtgtcct cctctagtgc ctcctcggta 3840
   ccttagggca acgccactgt gggggaaacg ggagagaact tgaaccacag gctcggcgga 3900
   ggaggccacc ctggggtaca acccctggtc ctctcacctg gatggctata ggcaagagtt 3960
   gcccatcggg ctgcagcttc agcatgacga ggggggcagc caggtactgc tgactacaaa 4020
   ggatgacatt ggccttgatc ccatccagaa ggaagaaatc cgcttcaaac agagtgcctt 4080
   tctgcatagg gagagaaatc aagggcttct gtcagcatgg tcttctccca ccctgagtgc 4140
   ccgacaagga gacaagaaag aagagagacg acctgtctcc atcagccacc tggcagtgtt 4200
   tgtcctctac ctcatcaaag tctcctcatt cttcttcctt tttttcttct tactttttta 4260
   ttgcatttgt ttgtttgctg ggtgggtgtg tatgttcgtg tatatgcata tatgtggaca 4320
   catgtttgcc atgacatcgg gtgatgatca gaagacaacg tatgggaagt agctccctcc 4380
   ttttaccaca caggtcccag ggatcaacta agattgttat gcttggcagc aggtagctct 4440
   acctggcgtt accacaccta accttatatg ctccgtctaa attactccag attacaggaa 4500
   aggaaaccga aagggtttcc cagaatcagg gtatcttggc aatcgggagc caaggaatac 4560
   cccaaagtca caccatgcaa caaacctcac acatgagatt tattggggtg acagaacggc 4620
   ggtggcctct gaacaggaac agagaaagca gcgagctgag tggaggggcg ggcttttaca 4680
   gggtctttgg agcatgtgca gccagcgagt ggagcatgcg cagtgagcta gtgaaacagt 4740
   acagagagcc tagaagtatt gcttgactgt gaaccttgaa taataggggt ggagatttcc 4800
   aagtcccgag ttcagggaca gaccaggggc agggtgattt tccactggtc tattacccta 4860
   aagaaacaga tcagagaaat gagatagtgc acttaaaatc acccagcaaa accaggattt 4920
   agatgcaagt ctatctggcc ccatgaatct tgtcattgtc ccagcttggt tcctaattag 4980
   ccccagccct gccttggatt ctctcaccct gtacctctct ggacccatgg cctccaggac 5040
   aacgtcacat ctctactggc caccccaata aggctcctct cttcaaccat atactcctct 5100
   cctgtgctgg gaaagccacg gtactacatt tttctacatc agtctaggat ttgtctggat 5160
   cctctcttga caaccagcag gaattcacag taaatctgag cacagtacac ccaacaccca 5220
   gtacacccaa cacccagtac acccaacacc cagtacaccc aacacccagt acacccaaca 5280
   cccagtacac ccaacaccca gtacacccaa cacccagtac acccagtaca cccaacaccg 5340
   gctcctgcct ccacttccct gcttggcctt cctggtgctt tgatgcccat accttgagct 5400
   cctcatccag ctgggcctgt agcttctcca tccccggagg gaataccagg cgggcaggga 5460
   gacaagtaga ccgcttcagc accatggggt tagcaccatt gagaaactgg tacccaaaga 5520
   aagcatcctc tttccaggag tttcgaaccc gctctaggga taggaggagg acgtgtctaa 5580
   gatccaactt cctggtcctt ccaacaccca ccaaaacatc cctacttgac ccagactacc 5640
   agagatgtca gacaaaagcc tctgacccac ccaccctcaa cccggctttc tcgatctcct 5700
   tgcattggga agagttttgg ggtaagagta atttgggggt aggtgtactt accagccatc 5760
   ttggtgtggc cactcttgaa aacatagttg aagtcatcta ggcttttcca gcaggtcaca 5820
   gtgtttttag ggaagttaat aacggtgtcc attgtccttc agggaagaat ggggcaaagg 5880
   atgtgacagt tatacttaga accaaacatt acagccaaag gtaccataga cgagaccagc 5940
   acacacccac cctcaagccg ggcactcttg ccctcacccc agaacctgtg aagcttcaaa 6000
   ttcaagtctt ttgtcctctc gaaatcgctg gtctacaggg aggtcagaga tactggtcgc 6060
   cgccacgttc aggattgtgc catccttcca gttgccccac ctgagggcag aagtgacatc 6120
   aggcacgggc actgcagagg cagctcgcca tgggtcccct gggtcagcct cacctgtaca 6180
   gactcctcct ttcttccagt tcctcctccc tgtggttcct gaacaggcct tgagagtctt 6240
   caaccacggt gcagcctaga agccgaggag ggagacactc tggatgtctt ctgtgagctt 6300
   ctctaccccc ttccagctgg ctgtacctct tcaggcagcc ctggctgtcc tctgtgcatg 6360
   catcttcatg tccctccctg aagcctccgt gacatctggg ccagcttcag ctactctttc 6420
   tttgacaact catttaaaac accaaacaaa cagcccagct tccaggctcc agctcccaca 6480
   cgctgcctcc ccttcctcac acaactgagg ctccgttccc cctatgcctc ttcacacaca 6540
   cacaccccac ccctctgctc accagtgccc tcagggaggt tcaggatgct ggtgccctga 6600
   acccatcggt aacaggggaa cgtgtactcc gatccctggt ctccggggcc cttcacagaa 6660
   atccagttgc agaaccaggc gtcctctttg agataatgcc atttctgcac tctcacaaac 6720
   agcagtggcc caaggtattc tgacacatcc accttgaatt ctgcctcctg cagataagca 6780
   caggggcgca gaatggggtg ctcaggctga gttctgcttc cactgggcca caggaaaagt 6840
   gatgaaaaga ggctccatga caccactcag ggttgtcacc tcaaccaaga gtgagagggt 6900
   taaggatgga cgatggacga tgggaaagta cactccttcc tccccggggg gtggggggag 6960
   gactagagga aggggaattc tggaaactct gccagaggcc cagtgaggaa cgggtcaaat 7020
   gggagacccc actaaggcag agaacccatg ggaattccat ttggcctgag ccagggtttc 7080
   tacaggatgg tctccaggga gaaacctgga tttggagagg cggggttggt gagagagaat 7140
   gaaatcagag aagacacgag gcagagtcca tggagagcgt agaggctgaa aggaccagcg 7200
   ccttcatgaa gctagggccc ccagcgcctt catgaagcta gggcccagag agccgtgggg 7260
   actgggagcc aaacaggacc aggtcccgag ggaccgagag taagccagag gcttggcagt 7320
   tcgtagggaa gaagctggag acactgggag gctttatcag gcagactgtt ctacagtagg 7380
   aacgagagaa gatggtaaat cttaagcttt aaaaaattag agacattcgg gcagctgaca 7440
   ctgctgatac tggcactact actgtaccgc agagttctgt gaagtgaggg acgcacagaa 7500
   atatcccact caatgaggcc aggaggcagc aactgttact gagcgctcac cgaatgcctc 7560
   tgtgcaccct ttccgctttc cgaggctgtg agctccttcc tcagagttca ggggccacgc 7620
   atcagggttc ctagggattt ctttgacttt gcccccgccc ccgcccccag cccctcaccc 7680
   tgccgcctcc cgggacagcc agagctctgc gcagctcacc gagttccgac agggtcggaa 7740
   cagcttcccg agagatgcct ccccatgctg tccgatcaac cacaggtaga cctcgttgtt 7800
   ggagcccgcg tacacggagt ccccggtgga gacgcggatg cggtagacac ccatcttgca 7860
   gatg 7864
<210> 2
   <211> 1557
   <212> DNA
   <213> Mus musculus
<220>
   <221> Mus musculus leukocyte-type 12-lipoxygenase gene
   <222> (1)..(1557)
   <223> GenBank accession no. U04332 as of 07 Jan 2003
<220>
   <221> SNP
   <222> (541)..(541)
   <223>
<400> 2
   gtgaaggtaa aaatcacaag cttggtacct cctgttgaaa tagttgtgat gcccattagc 60
   aagcaggaca ggataggaac gtttaagaag aatagggcac atttgtgtca ccatggcatt 120
   tctgtttgat tcgggagcag agagccttgc atttacagaa acagaacaat tcaggttagc 180
   gaacgttcca gtaagtacca tacgtgcctg agagtaatgc tgacctcagt tccaccggcg 240
   gtcgggtcag ggcttagcaa atatgtgatt cttatttcaa tggaaggggt gtgttagctt    300
   ggggatgtgc acagaactga ctcatagtgg agattgccat tcaccagaca cattttcatt    360
   gtctttgttt aattcagttt ataacactcc atgccagaat ggcaccaaag gtcattttat    420
   tactcttatt agtcacatgg actgagagtc tttgtggtcc catgtctccc tagaggaagg    480
   atgtatgtac agttgcattc agctcagtgg gttccaggtg tagcaacagt taacaataaa    540
   cgaataagcc ctgctggatg cggtcttggc ctaggaggcg ctcctgtgag ccggtgatac    600
   tgacacaagg atgatcaggg tccagcatta gccaggcatc agcagaagcg cttctggggt    660
   aggcggtaga agctggttgt ctctgggtgg ggtgtagaag gcacttcact gagaagctag    720
   agtctcctca ggatttggaa ggatgagtag ggtggacccc gagtacatga ttcttcagca    780
   aataacatgg agtctttggt ggtggctctg gggagcaagg ggcttcagca ggagtgcagt    840
   gtggggaagg gagaaaatca ggtttccgag ggctttgaag gccaggtaat ttcgggtttt    900
   attatgcaga catcgggaga tatctggatg ctttgaaact cacagggatg agcccagagc    960
   ctgcctcagg atgggcaaag tgtacagagc tggtagaatt aatcgggtgg ctaaaatgtg   1020
   ccgcctttaa atgctgcaca gcacctgcar ccttaagatt atccctgggc atgggaaaac   1080
   ccatctgtga tgcgcgcact ggagagtgga tgtatgtgcg tgtgagagtg tgtgtgtgtg   1140
   tgtgtgtgtg tgtgtgtgtg tgtgagagag agagagagag agagagagag agagagagag   1200
   agagagagag agagtgagcg cgcgtggagg aaggtgtgac gggggcagaa gggactggac   1260
   cagatgttga ttctttcttg catgttttct tttcaaatcc ccatctactt ctccctcctg   1320
   ttctcctctc accaagatga tttttgagag ttcagctttg cccagagccc cgcctttttt   1380
   gggctggaac gaggccccca gcaagtgctg ggggccccag taagtgacag aggcggggtt   1440
   aattgccacc tacagctcaa ccctaaacta gacttctgaa ggggcggggc ctcggctttc   1500
   ttatttagcc cgtctacccc tcttgattct cagaagggag aaaacttcat ctgcaag      1557